(19) Europäisches Patentamt
European Patent Office
Office européen des brevets

(11) Publication number: 0 137 729
B1

(12) **EUROPEAN PATENT SPECIFICATION**

(45) Date of publication of patent specification: **30.08.89**

(51) Int. Cl.$^4$: **A 01 N 37/22**

(21) Application number: **84306148.2**

(22) Date of filing: **07.09.84**

# (54) Method for the control of fungal growth in paints or cutting fluids.

(30) Priority: **28.09.83 US 536527**

(43) Date of publication of application:
**17.04.85 Bulletin 85/16**

(45) Publication of the grant of the patent:
**30.08.89 Bulletin 89/35**

(84) Designated Contracting States:
**DE FR GB IT NL**

(56) References cited:
**EP-A-0 010 166**
**EP-A-0 100 615**
**DE-C- 834 152**
**FR-A-1 546 820**
**FR-A-2 336 079**
**GB-A-1 401 975**
**US-A-3 781 442**

**CHEMICAL ABSTRACTS, vol. 49, no. 3, 10th February 1955, column 1611, Columbus, Ohio, US; N.G. CLARK et al.: "The fungicidal activity of substituted acetanilides and related compounds"**

(73) Proprietor: **COSAN CHEMICAL CORPORATION**
**400 Fourteenth Street**
**Carlstadt New Jersey 07072 (US)**

(72) Inventor: **Gaglani, Kamlesh**
**1998 Second Place**
**South Plainfield New Jersey 07080 (US)**
Inventor: **Eilender, Albert L.**
**6 Peach Tree Drive**
**Montville New York 07045 (US)**

(74) Representative: **McCallum, William Potter et al**
**Cruikshank & Fairweather**
**19 Royal Exchange Square**
**Glasgow G1 3AE Scotland (GB)**

(56) References cited:
**CHEMICAL ABSTRACTS, vol. 47, no. 20, 25th October 1953, columns 10805,10806, Columbus, Ohio, US; H. OZAWA et al.: "Chemotherapy against trichophyton. I. Relationship between chemical structures of haloacyl derivatives of aromatic primary amines and their fungicidal activities"**

**CHEMICAL ABSTRACTS, vol. 52, no. 11, 10th June 1958, column 9507, Columbus, Ohio, US**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

CHEMICAL ABSTRACTS, vol. 50, no. 1, 10th January 1956, column 209, Columbus, Ohio, US; D. BEKE et al.: "New local anesthetics"

CHEMICAL ABSTRACTS, vol. 86, no. 21, 23th May 1977, page 463, no. 155556k, 10th coll. index, page 124CS, Columbus, Ohio, US; Y.V. SVETKIN et al.: "C-(arylcarbamoyl)-N-phenylnitrones"

CHEMICAL ABSTRACTS, vol. 67, no. 17, 23th October 1967, page 7602, no. 80804m, 8th coll. index, page 625, Columbus, Ohio, US; A. BOROVANSKY et al.: "A study in local anesthetics. XXXI. Basic 4-bromo-2,6-dimethylacylanilines"

CHEMICAL ABSTRACTS, vol. 89, no. 23, 4th December 1978, page 590, no. 197117k, 10th coll. index, page 136CS, Columbus, Ohio, US; W. PRINZ et al.: "A novel synthetic route to oxominoacet-2-anisidides and their conversion into 3-hydroxyanthranilic acids via 7-methoxyisatins"

**Description**

The invention relates to processes for preparing N-substituted alpha-chloroacetamides which are useful as fungicides. This invention further relates to methods of controlling or modifying the growth of fungi in certain industrial fluids.

The term "fungicide" as used herein and in the appended claims means materials having a modifying effect upon the growth of fungi. Such modifying effects include all deviations from natural development for example, killing, retardation, inhibition, and the like. The term is also used to identify the growth modifying activity of the compounds and compositions of this invention. A fungicide is commonly used to prevent either the destructive action or the unsightly appearance of fungi (or the enzymes produced during their growth) on materials by killing or inhibiting the growth of these organisms.

The term "fungi" as used herein and in the appended claims means any nucleated, usually filamentous, sporebearing organism devoid of chlorophyll. The common term for fungi encountered in industrial biodeterioration in the non-scientific community is molds and mildews. Molds are defined as any of the small, multinucleate, possibly multicellular, filamentous fungi included in the *Eumycetes*. Mildew is the term usually applied to the growth of fungi on substrates such as fibers, fabrics, plastics, masonry and wood and results in discoloration and sometimes weakening or even disintegration of the material on which the growth occurs. Because more than a hundred species are involved in mildew, it has become a generic term indicating growth producing discoloration of the material with or without degradation of the substrate.

The term "industrial material" as used herein refers to all products or substances other than foodstuffs and living things. Thus it includes paper, wood, paint, rubber, plastics, etc., but excludes food and drink, plants and animals and their vital parts or products. By extension, it includes those fungi which are pathogenic, or phytopathogenic, which therefore excludes the fungi which are cited in the agricultural and/or insect literature and application patents.

An object of this invention is to provide a process for the control of fungal growth by the use of fungicidal compositions containing as an essential ingredient an N-substituted alpha-chloroacetamide having one, two or three ring substituents.

The invention provides a method for the control of fungal growth in aqueous liquid latex paints or metal cutting fluids which comprises adding to such latex paints or metal cutting fluids a fungicidally effective amount of a compound of the formula

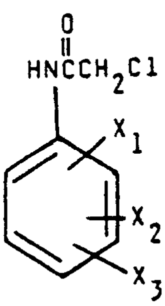

wherein $X_1$, $X_2$ and $X_3$ are moieties each selected from the group consisting of hydrogen, halogen, and lower alkyl.

Preferably the lower alkyl is methyl or ethyl. The preferred halogen is a fluoride, a chloride or a bromide.

The preferred compounds for use in the method are selected from the group consisting of
N-(3,4-dichlorophenyl)-2-chloroacetamide,
N-(4-chloro-2-methylphenyl)-2-chloroacetamide,
N-phenyl-2-chloroacetamide,
N-(2-bromophenyl)-2-chloroacetamide,
N-(4-bromo-2,6-dimethylphenyl)-2-chloroacetamide,
N-(2,4-dibromophenyl)-2-chloroacetamide,
N-(2,6-dibromo-4-methylphenyl)-2-chloroacetamide, and
N-(4-bromo-2-methylphenyl)-2-chloroacetamide.

The alpha chloroacetamides for use in the method of the invention are formulated for sale as antifungal compositions with inert organic solvents such as dimethylformamide, N-methyl-2-pyrrolidone, 2-methoxy ethanol, 2-ethoxy eithanol, triethylphosphate, tributylphosphate and the like. The antifungal concentration of the compounds for use in the method of this invention in such compositions ranges from about 10 to 60% by weight. The user employs the antifungal compositions in various environments in ranges from about 500 to about 30,000 ppm, preferably 5,000 to 15,000 ppm.

Although recent years have witnessed a large number of new microbicidal agents, there is still a need for fungicidal agents suitable for use in industrial environments to inhibit biodeterioration. Most industrial

antifungal compounds were developed specifically for agricultural use. However, the requirements for an antifungal agent in agriculture are vastly different from those required in industrial biodeterioration. In order for the agricultural fungicides to be commercially successful, not only is their fungicidal activity of importance, but a minimal phytotoxicity and mammalian toxicity are required. An antifungal agent designed for agricultural use must have a relatively short half-life and must break down in the environment into non-toxic chemicals. In the field of industrial biodeterioration the phytotoxicity of a fungicide is of no importance; the mammalian toxicity is of secondary consideration; and, most importantly, long term stability and lack of chemical breakdown are highly desirable and sought after characteristics. Thus, the requirements for an industrial antifungal agent are quite different from an agricultural antifungal agent. Compounds having somewhat similar structures to the present invention are known to be effective for control of fungal organisms indigenous to the plant (agricultural) community.

However, those disclosed compounds are not at all suitable for use in an industrial environment. For industrial use, the compound must be stable, *i.e.,* it must retain its antimicrobial effects over a long period of time under relatively harsh conditions. Also, the compound must not affect the particular industrial process to which it is applied. Phytotoxicity is of no consequence and the mammalian toxicity is of minimal importance in the overall selection of a suitable compound.

For example, coatings such as paints are often exposed to environments with high moisture content and warm temperatures which favor mold formation. Under such conditions, fungal growth can occur to such an extent that the paint can discolor and turn dark. In some instances, the fungal growth can cause decomposition of the paint binder and a concommitant loosening of the film. Also the paint may be loosened and detached from the substrate. Emulsion latex paints are particularly prone to fungal attack. These emulsion type lattices contain polymers more likely to be attacked by fungi than alkyd resin-based paints which have a greater concentration of aromatic rings. United States Patent No. 3,505,457 discloses 2,5 disubstituted alpha-iodoacetanilides, compounds to some extent structurally similar to the present invention. The compounds disclosed have an alpha-iodide in place of the alpha-chloride of the present invention. Although these alpha-iodoacetanilides do have fungicidal activity and are acceptable for use in the agricultural community, they are completely unsuitable for industrial fungicidal purposes. The alpha-iodoacetanilide is easily hydrolyzed under mild environmental conditions into its component parts alpha-iodo-acetic acid and the 2,5-disubstituted aniline. There is no fungicidal activity after hydrolysis. In contrast, the present invention, the alpha-chloroacetamides, are not easily hydrolyzed under normal environmental conditions and therefore have much longer half-lives and retain their antifungal activity longer. The alpha-chloroacetamides will therefore retain their anti-fungal activity over a much longer time than will the compounds described in the prior art.

It should be noted that the alpha-bromoacetamides have similar chemical properties as the iodo analogs (i.e., will hydrolize under mild conditions) and are therefore not suitable for use in industrial environments.

An industrial fungicide must also not affect the industrial material with which it is mixed. For example, when above mentioned iodoacetamides (e.g., N-(5-chloro-2-methoxyphenyl)-2-iodoacetamide and N-phenyl-2-iodoacetamide are mixed with paint, the paint forms a thick gel after 24 hours and is unusable. The corresponding alpha-bromoacetamides also cause gelling of paint after twenty-four hours.

Thus, the compounds that are used in the method according to the present invention are not only different structurally from the compounds cited in the prior art but also have surprisingly different properties making these compounds uniquely suitable for inhibiting industrial biodeterioration.

Industrial biodeterioration is an extremely broad problem and encompasses, but is not limited to, the following categories of industrial materials: paint, adhesives, floor polishes and finishes, pigments, colorants, and extender slurries, building materials such as joint cements, tape muds, acoustical plasters, tub caulks and grouts, cutting fluids, secondary oil recovery, specialty wood preservatives, cosmetics, textiles including fabrics and floor coverings, cooling towers, pulp and paper slimicides.

The alpha-chloroacetamides of this invention are effective in the control of fungi in industrial materials. The present compounds exhibit microbiocidal action on a wide variety of fungi such as *Penicillium sp, Aspergillus niger* and other sp, *Alternaria solani,* and others, *Aureobasidium pullulans, Cladosporium resinae,* and *Fusarium sps.*

The chloroacetamides described can be prepared by chloroacetylation of suitable nuclear-substituted aromatic amines. The chloroacetylating agent is preferably either a chloroacetic anhydride or a chloroacetyl halide such as chloroacetyl chloride. The choices of chloroacylating agent is determined to some extent by the nature of the ortho substitution of the aromatic amine to be acetylated. Thus the chloroacetic anhydride is ordinarily used with a difficultly acetylated amine such as 2,6-di-tert-butyl amine but it is usually preferred to use a chloroacetyl halide to acetylate the more reactive amines.

The chloroacetylation reaction is preferably conducted in the presence of a suitable organic medium. The organic medium must be anhydrous if the acetylating agent is a chloroacetic anhydride. However, either anhydrous media or media containing water can be used with chloroacetyl halide acetylating agents. Suitable organic media for use with either acetylating agent include, for example, benzene, diethyl ether, 1,2-dichloroethane, hexane, chlorobenzene, toluene, chloroform, and the xylenes.

The chloroacetylation reaction is generally carried out at a temperature which is between 90° and 115°C. It is generally preferable to carry out the reaction at atmospheric pressure although sub-atmospheric

pressure and super-atmospheric pressure can be used. The acetamide products may be separated from the reaction mixture by methods well known to those skilled in the art, such as by distillation or fractional crystallization from the reaction medium or from solvents in case the desired product is a soluble substance.

The fungicidal activity of the alpha chloroacetamides was determined by measuring the minimum inhibitory concentration (MIC). The MIC of the various compounds is the lowest amount of a biocide which will inhibit the growth of a microorganism or a mixture of microorganisms. It consists of a series of test tubes containing a growth medium for the organism(s) of interest: bacteria, fungi, etc. The compound is measured into the medium by serial dilution in an exact series of steps, often a decimal series, 1, 2, 3, 4 etc., or 10, 20, 30 etc., and after inoculation and a suitable incubation, examined to determine the minimum amount of the compound which prevented growth. The inhibitory concentration is reported as the lowest amount, in parts per million, showing no growth when examined by the unaided eye. In tests where the medium is initially turbid, a small sample from each vessel is placed onto Nutrient Agar in Petri dishes and after 24 hours, examined for growth. Again, the lowest concentration in parts per million of compound showing no growth is reported. The test organism for determining the MIC for fungi is a week old culture of *Aureobasidium pullulans* (the most common mildew fungus). A summary of the MIC's of the preferred embodiments of the present invention is presented in Table I.

TABLE I

| Compound | MIC |
|---|---|
| N-(2,4-dichlorophenyl)-2-chloroacetamide | 31 |
| N-(2-methoxyphenyl)-2-chloroacetamide | 125 |
| N-(3,4-dichlorophenyl)-2-chloroacetamide | 31 |
| N-(4-chloro-2-methylphenyl)-2-chloroacetamide | 62 |
| N-(2-methylphenyl)-2-chloroacetamide | 250 |
| N-(4-bromo-2-methylphenyl)-2-chloroacetamide | 8 |

The advantages, desirability and usefulness of the present invention are illustrated in the following examples.

Example 1

In this example, N-(3,4-dichlorophenyl)-2-chloroacetamide was prepared by adding 113 grams of chloroacetylchloride in 80 cc of toluene to 165.3 grams 3,4-dichloroaniline and 950 cc toluene at 25°—30°C over one hour. After the addition of the chloroacetylchloride was complete, the temperature of the reaction mixture was raised to reflux temperature (approximately 115°C) and refluxed for 16 hours. The reaction mixture was then cooled to 0°C and the precipitated product was separated by filtration. The precipitated product was then recrystallized resulting in a product that weighed 176.9 grams and having a melting point of 104°—105°C.

Example 2

In this example, N-(4-bromo-2-methylphenyl)-2-chloroacetamide was prepared by adding 60 grams of chloroacetylchloride in 50 cc of 1,2-dichloroethane to 100 grams 4-bromo-2-methylaniline and 600 cc 1,2-dichloroethane at 25°—30°C over one hour. After the addition of the chloroacetylchloride was complete, the temperature of the reaction mixture was raised to reflux temperature (approximately 85°C) and refluxed for 25 hours. The reaction mixture was then cooled to 0°C and the precipitated product was separated by filtration. The precipitated product was then recrystallized resulting in a product that weighed 123 grams and having a melting point of 133°—135°C.

Example 3

In this example, N-phenyl-2-chloroacetamide was prepared by adding 22.6 grams of chloroacetylchloride in 16 cc of Toluene to 18.6 grams aniline in 107 cc Toluene at 25°—30°C over one hour. After the addition of the chloroacetylchloride was complete, the temperature of the reaction mixture was raised to reflux temperature (approximately 115°C) and refluxed for 16 hours. The reaction mixture was then cooled to 0°C and the precipitated product was separated by filtration. The precipitated product was then recrystallized resulting in a product that weighed 31.1 grams and having a melting point of 133°C—135°C.

Example 4

In this example, N-(2-bromophenyl)-2-chloroacetamide was prepared by adding 16.1 grams of chloroacetylchloride in 15 cc of Toluene to 25 grams 2-bromoaniline and 150 cc Toluene at 25°—30°C over one hour. After the addition of the chloroacetylchloride was complete, the temperature of the reaction mixture was raised to reflux temperature (approximately 115°C) and refluxed for 16 hours. The reaction mixture was then cooled to 0°C and the precipitated product was separated by filtration. The precipitated product was then recrystallized resulting in a product that weighed 22.7 grams and having a melting point of 76°C—77°C.

Example 5

In this example, N-(2,4-dibromophenyl)-2-chloroacetamide was prepared by adding 11.3 grams of chloroacetylchloride in 20 cc of Toluene to 25 grams 2,4-dibromoaniline and 150 cc Toluene at 25°—30°C over one hour. After the addition of the chloroacetylchloride was complete, the temperature of the reaction mixture was raised to reflux temperature (approximately 115°C) and refluxed for 16 hours. The reaction mixture was then cooled to 0°C and the precipitated product was separated by filtration. The precipitated product was then recrystallized resulting in a product that weighed 29.7 grams and having a melting point of 107°C—108.5°C.

Example 6

The compounds were tested as to their ability to inhibit the growth of fungi (mildew) of a paint film. The method uses a small environmental chamber constructed so as to maintain both the temperature and humidity at optimal conditions for the growth of fungi involved in mildew growth. A tray of soil is inoculated with mildew fungi and placed in the bottom of the cabinet over approximately 1 inch of water; the closed cabinet is maintained in a 25°C incubator. The organisms used are *Aureobasidium pullulans* (ATCC9348), *Aspergillus niger* (ATCC6275) and *Penicillium sp.* (ATCC9549). The fungal growth in the soil provides a constant air-borne inoculation for the surface of panels exposed in the top of the chamber.

The panels are made of clear, white pine sapwood 37.5 mm × 75 mm × 9.4 mm. Each panel is coated twice with a standard paint containing the fungicide at the desired concentrations. Panels coated with the paint containing no fungicide are used as the negative control. The painted panels are hung vertically from the top of the chamber with the bottom of the panel approximately 3 inches above the soil tray. Replicate panels are placed randomly in the chamber. The panels are examined and rated each week for six weeks. Untreated painted panels fail in two to three weeks. The panels are rated from 10 (no growth) to 1 (maximum growth).

This method is described in Federal Specification TTP29H and is that of ASTM D3273—73T.

TABLE II

Growth Control with Data at Indicated Week Intervals

| | Weeks | | | |
|---|---|---|---|---|
| | 1 | 2 | 4 | 6 |
| Control — no fungicide | 10* | 9 | 4 | 1 |
| N-(3,4-dichlorophenyl)-2-chloroacetamide | 10 | 10 | 10 | 9 |
| N-(4-bromo-2-methylphenyl)-2-chloroacetamide | 10 | 10 | 10 | 10 |
| N-phenyl-2-chloroacetamide | 10 | 10 | 7 | 5 |
| N-(2-bromophenyl)-2-chloroacetamide | 10 | 10 | 7 | 5 |

* 10 = no growth; 1 = maximum growth

Example 7

The efficacy of the present invention in inhibiting fungal growth in metal cutting fluids (both soluble oil and synthetic cutting fluids) was tested. Even in this industrial area of biodeterioration known to be primarily subject to bacterial spoilage, occasional fungicides are required. So, even though the products of the instant invention are not effective bactericides, their fungicidal activity is valuable and easily demonstrated. This procedure evaluates the effectiveness of a biocide to control bacteria and/or fungi responsible for the deterioration of metal working fluids. The antimicrobial agent is incorporated into a metal-working fluid at its use concentration (1000 parts per million). The mixture is then inoculated with microbial culture (fungi and bacteria) known to degrade such fluids. The fluid is aerated for five (5) days and then is unaerated for two days, thus simulating normal plant operations. The fluid is sampled before and

after aeration and the samples evaluated for pH, total fungal and bacterial count. This regimen is continued until the antimicrobial fails by allowing a bacterial count of 1 × $10^7$ organisms per ml or a duration of six weeks of limited growth occurs. This follows the ASTM Method E 686—80.

The organisms used are either a culture of bacteria and fungus which normally would contaminate a cutting fluid: *Staphylococcus aureus, Flavobacterium species, Pseudomonas aeruginosa, Escherichia coli, Desulfovibrio desulfuricans,* and the fungi *Cladosporium resenae* and *Fusarium sp.* In all cases where bacteria were the inoculum, there was an overgrowth of 1 × $10^7$ organisms in the negative control by the end of the first week. The results of the fungicidal tests are summarized in Table III.

TABLE III

| | Weeks | | | | | |
|---|---|---|---|---|---|---|
| | 1 | 2 | 3 | 4 | 5 | 6 |
| Soluble Oil | | | | | | |
| Control | 9* | 28 | 39 | 57 | 59 | TNTC** |
| N-(3,4-dichlorophenyl)-2-chloroacetamide | 7 | 3 | 0 | 0 | 0 | 0 |
| N-(4-bromo-2-methylphenyl)-2-chloroacetamide | 2 | 1 | 1 | 0 | 0 | 0 |
| N-(5-chloro-2-methoxyphenyl)-2-chloroacetamide | 3 | 1 | 2 | 0 | 0 | 0 |
| N-phenyl-2-chloroacetamide | 0 | 0 | 0 | 0 | 0 | 0 |
| N-(2-bromophenyl)-2-chloroacetamide | 0 | 0 | 0 | 0 | 0 | 0 |
| N-(2,4-dibromophenyl)-2-chloroacetamide | 0 | 2 | 4 | 0 | 0 | 1 |
| Synthetic Cutting Fluid | | | | | | |
| Control | 7 | 61 | 46 | 47 | 180 | 400 |
| N-(3,4-dichlorophenyl)-2-chloroacetamide | 7 | 0 | 0 | 0 | 0 | 0 |
| N-(4-bromo-2-methylphenyl)-2-chloroacetamide | 10 | 0 | 0 | 0 | 0 | 0 |
| N-(5-chloro-2-methoxyphenyl)-2-chloroacetamide | 15 | 0 | 0 | 0 | 0 | 0 |
| N-phenyl-2-chloroacetamide | 10 | 0 | 0 | 0 | 0 | 0 |
| N-(2-bromophenyl)-2-chloroacetamide | 0 | 0 | 0 | 0 | 0 | 0 |
| N-(2,4-dibromophenyl)-2-chloroacetamide | 1 | 2 | 7 | 3 | 3 | 1 |

* The data represent total organism count
** Too numerous to count

The data available with respect to the bacterial counts of the compounds listed in Table III indicate that the compounds of this invention are not effective bactericides. More specifically the data obtained demonstrates that the compounds of this invention exhibit positive fungicidal activity and little, if any bactericidal activity.

## Example 8

The fungicidal compounds were examined to determine whether they could prevent the growth of a cellulose degrading fungus on a chip of treated wood.

Thin chips of transversely cut clear white pine about 40 mm × 40 mm × ½ mm are weighed, dipped into a solution of known concentration of the fungicide, dried and reweighed to determine the retention of the compound. The treated chips are placed on a glass "spacer" 5 mm above a fully developed growth of the fungus *Coniophora puteana Duby* (ATCC36336) on a plastic petri dish of Malt Extract Agar. To prevent drying, the petri dishes are kept in polyethylene bags and are incubated at 25°C for four weeks. In this time, an untreated control is heavily deteriorated. Samples which are sufficiently protected do not show any significant traces of fungal attack.

TABLE IV

Sutter Test

| | Retention Weight (grams) | Growth Reported in % Coverage Weeks 1 | 2 | 3 | 4 |
|---|---|---|---|---|---|
| Control | | 1 | 7 | 80 | 100 |
| N-(3,4-dichlorophenyl)-2-chloroacetamide | 0.06 | 0* | 0 | 3 | 12 |
| | 0.084 | 0 | 1 | 1 | 1 |
| | 0.08 | 0 | 0 | 0 | 0 |
| N-(5-chloro-2-methoxyphenyl)-2-chloroacetamide | 0.105 | 0 | 0 | 0 | 0 |
| | 0.10 | 0 | 0 | 0 | 0 |
| | 0.10 | 0 | 0 | 0 | 0 |
| N-(4-bromo-2-methylphenyl)-2-chloroacetamide | 0.06 | 0 | 0 | 0 | 0 |
| | 0.085 | 0 | 0 | 0 | 0 |
| | 0.08 | 0 | 0 | 0 | 0 |

* 0 = no growth; 100 = 100% coverage of mildew on the test specimen

While in the foregoing specification there have been set forth specific embodiments of this invention for purposes of illustration, it will be apparent to those skilled in the art that the specific embodiments and details thereof can be varied widely without departing from the spirit and scope of this invention.

**Claims**

1. A method for the control of fungal growth in aqueous liquid latex paints or metal cutting fluids which comprises adding to such latex paints or metal cutting fluids a fungicidally effective amount of a compound of the formula

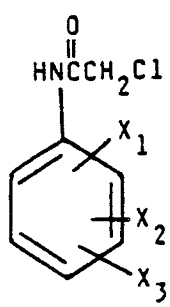

wherein $X_1$, $X_2$ and $X_3$ are moieties each selected from the group consisting of hydrogen, halogen, and lower alkyl.

2. A method according to claim 1 wherein the compound is present in an amount of from about 500 to 30,000 ppm.

3. A method according to claim 1 or claim 2 wherein the lower alkyl is methyl or ethyl.

4. A method according to claim 1 or claim 2 wherein the halogen is a fluoride, a chloride or a bromide.

5. A method according to claim 1 or claim 2 wherein the compound is selected from the group consisting of
N-(3,4-dichlorophenyl)-2-chloroacetamide,
N-(4-chloro-2-methylphenyl)-2-chloroacetamide,
N-phenyl-2-chloroacetamide,

N-(2-bromophenyl)-2-chloroacetamide,
N-(4-bromo-2,6-dimethylphenyl)-2-chloroacetamide,
N-(2,4-dibromophenyl)-2-chloroacetamide,
N-(2,6-dibromo-4-methylphenyl)-2-chloroacetamide, and
N-(4-bromo-2-methylphenyl)-2-chloroacetamide.

**Patentansprüche**

1. Ein Verfahren zur Kontrolle des Pilzwachstums in wässerigen flüssigen Latex-Anstrichmitteln oder Metallborflüssigkeiten, welches den Zusatz einer effektiven pilztötenden Menge einer Zusammensetzung der Formel

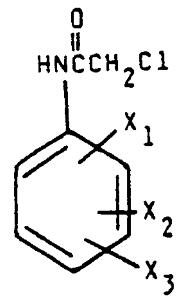

zu besagten Latex-Anstrichmitteln oder Metallborflüssigkeiten umfaßt, wobei $X_1$, $X_2$ und $X_3$ Teile sind, die jeweils von der aus Wasserstoff, Halogen und niedrigem Alkyl bestehenden Gruppe ausgewählt werden.

2. Ein Verfahren entsprechend dem Anspruch 1, worin die Zusammensetzung aus einer Menge zwischen etwa 500 bis 30.000 ppm besteht.

3. Ein Verfahren entsprechend dem Anspruch 1 oder Anspruch 2, worin das niedrige Alkyl entweder Methyl oder Äthyl ist.

4. Ein Verfahren entsprechend dem Anspruch 1 oder Anspruch 2, worin das Halogen entweder ein Fluorid, ein Chlorid oder ein Bromid ist.

5. Ein Verfahren entsprechend dem Anspruch 1 oder Anspruch 2, worin die Zusammensetzung aus einer Gruppe ausgewählt wird, die aus folgendem besteht:
N-(3,4-Dichlorophenyl)-2-Chloroacetamid,
N-(4-Chloro-2-Methylphenyl)-2-Chloroacetamid,
N-Phenyl-2-Chloroacetamid,
N-(2-Bromophenyl)-2-Chloroacetamid,
N-(4-Bromo-2,6-Dimethylphenyl)-2-Chloroacetamid,
N-(2,4-Dibromophenyl)-2-Chloroacetamid,
N-(2,6-Dibromo-4-Methylphenyl)-2-Chloroacetamid, und
N-(4-Bromo-2-Methylphenyl)-2-Chloroacetamid.

**Revendications**

1. Procédé pour maîtriser la croissance de champignons ou moisissures dans des peintures-émulsions aqueuses liquides (peintures à base de latex) ou dans des fluides pour la coupe de métaux, ce procédé comprenant l'addition, à de telles peintures-émulsions ou à de tels fluides pour la coupe de métaux, d'une quantité, efficace du point de vue fongicide, d'un composé de formule

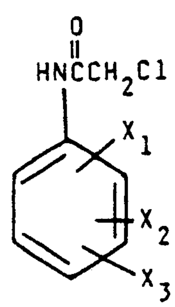

dans laquelle $X_1$, $X_2$ et $X_3$ sont des fragments choisis chacun dans l'ensemble constitué par un atome d'hydrogène, un atome d'halogène et un groupe alkyle inférieur.

2. Procédé selon la revendication 1, dans lequel le composé est présent en une quantité comprise entre

environ 500 et 30000 ppm ou millionièmes.

3. Procédé selon la revendication 1 ou la revendication 2, dans lequel le groupe alkyle inférieur est un groupe méthyle ou éthyle.

4. Procédé selon la revendication 1 ou la revendication 2, dans lequel l'halogène est un atome de fluor, un atome de chlore ou un atome de brome.

5. Procédé selon la revendication 1 ou la revendication 2, dans lequel le composé est choisi dans l'ensemble constitué par
le N-(3,4-dichlorophényl)-2-chloro-acétamide,
le N-(4-chloro-2-méthylphényl)-2-chloro-acétamide,
le N-phényl-2-chloro-acétamide,
le N-(2-bromophényl)-2-chloro-acétamide,
le N-(4-bromo-2,6-diméthylphényl)-2-chloro-acétamide,
le N-(2,4-dibromophényl)-2-chloro-acétamide,
le N-(2,6-dibromo-4-méthylphényl)-2-chloro-acétamide, et
le N-(4-bromo-2-méthylphényl)-2-chloro-acétamide.